# EUROPEAN PATENT SPECIFICATION

(11) **EP 1 535 064 B1**
(45) Date of publication and mention of the grant of the patent: **29.08.2007**
(21) Application number: 03762822.9
(22) Date of filing: 09.07.2003
(51) Int. Cl.: G01N 33/50, B01L 3/00, C12Q 1/04

(54) **MONITORING OF CELLS**
ÜBERWACHUNG VON ZELLEN
SURVEILLANCE DE CELLULES

(30) Priority: 09.07.2002 GB 0215879
(43) Date of publication of application: 01.06.2005
(73) Proprietor: Smart Holograms Limited, Milton Road Cambridge CB4 0WF (GB)
(72) Inventor: LOWE, Christopher Robin, Institute of Biotechn., Cambridge CB2 1QT (GB); GERSHATER, Craig J., Cambridge Biopr. Manag. Ltd., Cambridge CB2 5QJ (GB); DAVIDSON, Colin, Alexander, Bennett, Tennis Court Rd., Cambridge CB2 1QT (GB)
(74) Representative: Perry, Robert Edward
(86) International application number: PCT/GB2003/002976
(87) International publication number: WO 2004/005922

(56) References cited:
- WO-A-99/47922
- WO-A-99/67639
- US-A- 5 308 757
- MCCONNELL H M ET AL: "THE CYTOSENSOR MICROPHYSIOMETER: BIOLOGICAL APPLICATIONS OF SILICONTECHNOLOGY" SCIENCE, AMERICAN ASSOCIATION FOR THE ADVANCEMENT OF SCIENCE,, US, vol. 257, no. 5078, 25 September 1992 (1992-09-25), pages 1906-1912, XP000560386 ISSN: 0036-8075 cited in the application

## Description

### Field of the Invention

This invention relates to a method for observing or monitoring cells, in particular their metabolism, to optimising cell culture conditions, and for high throughput screening, and to a device suitable for use in such a method.

### Background of the Invention

A microfluidic device typically comprises at least one channel or chamber having a size that is equal to or less than 1 mm in at least one dimension. Of particular interest are microfluidic devices suitable for carrying out and/or monitoring chemical and biological processes. These devices can be used to simulate large-scale processes at a microscopic level, thus minimising volumes of fluids and reagents.

A microfluidic device may be used to monitor cell metabolism. Changes in the external biological, chemical and physical milieu of a cell specifies particular patterns of up- and down-regulated gene expression and results in changes in the concentrations and fluxes of metabolites within the cellular environment. All living cells take in nutrients and break them down to produce useful energy and waste products. For example, almost all cells change the pH of their environment by taking up alkaline or acidic nutrients or producing organic acids, CO₂ or NH₄⁺. Cell metabolism of this type can be monitored with a silicon-based microphysiometer, which measures the rate at which eukaryotic cells change the pH of their immediate environment in real time in thermoregulated microvolume flow chambers; see Wada et al, 1991, Clin. Chem. 37:600-601; and McConnell et al, 1992, Science 257:1906-1912.

This microphysiometer suffers from a number of limitations. For example, the light-addressable potentiometric sensor (LAPS), on which the system is based, relies on expensive microengineered silicon technology and has a relatively high microvolume (multiple µL). Furthermore, the device requires sophisticated instrumentation, uses biohostile inorganic surfaces and, as currently configured, is able to monitor only a single analyte (pH).

Conventional cellular microfluidic devices comprise channels and chambers having a relatively large volume (of the order of µL). The size of a microfluidic device is preferably as small as possible, so that reagent/sample quantities are minimised.

Since microfluidic technology deals with tiny volumes of reagents and fluids, the observation and monitoring of cells may depend critically on the surface area/volume ratio of the microfluidic chambers and channels, and on the nature of the biofilm that forms on the inner surface of a channel or chamber. Conventional microfluidic channels and chambers are typically restricted to volumes of the order of µL. If the volume is smaller (say of the order of nL), then the reduction in volume is not mirrored by a reduction in surface area and the effect of uneven biofilm formation is more pronounced.

The size of a microfluidic device may also be limited by the size of any sensor used. Other problems faced by microfluidic technology include the provision of low-cost devices and microfluidic circuits, the establishment of high-throughput methods for inoculation and the real-time detection of cellular activity.

Conventional microfluidic devices are generally fabricated in glass, silica or silicon-based substrates using photolithographic processing techniques to generate channels, reaction chambers or arrays. Whilst successful prototype devices have been prepared using these substrates, the associated fabrication techniques, including mask production, channel etching and sealing, and bonding and packaging, are generally difficult and/or expensive to implement. The large-scale production of micromachined devices is hampered by the fact that wafer sizes are relatively small, the cost of processing is prohibitive, set-up, tooling, design changes and revisions are expensive and the introduction of three dimensionality into the design requires expensive registration and etching or cutting facilities.

### Summary of the Invention

According to one aspect of the invention, cells are monitored in a microfluidic device comprising a sensor according to the claims. This is done under conditions such that attachment of cells to a wall of the device is inhibited.

According to another aspect of the invention, a microfluidic device comprises a chamber including a sensor and inlets for a sample and for a growth medium according to the claims. A wall of the device is such that, in use, attachment of cells to the wall is inhibited.

The chamber is preferably in connection with a second, downstream chamber. The second chamber may be used to detect cellular components such as expressed proteins or enzymes. In this case, a device of the invention may be considered to comprise a biotic (first) chamber in connection with an abiotic (second) chamber.

By inhibiting attachment of the cell to a wall of the chamber, biofilm formation is minimised or inhibited. The size of the microfluidic chamber can be less critical than in conventional microfluidic devices, despite having a high surface area/volume ratio, because the effects of biofilm formation are markedly reduced even at small volumes (e.g. of the order of nL).

A device of the invention preferably comprises a plurality of microfluidic chambers, which may be connected by microfluidic channels. Such an array may be used to study multi-factorial effectors such as carbon/nitrogen sources, vitamin and mineral supplements and their effect on microbial physiology.

A device of the invention may provide a rapid low-cost method of investigating the performance of whole cell systems and may be utilised in areas such as pharmaceuticals and biotechnology (whole cell screening, drug discovery, genomics, proteomics and metabolomics, tissue culture, biotransformations, chiral drug production), food and beverage (growth conditions, media optimisation, fermented products such as amino acids, vitamins, gums, acids), environmental (bioremediation) and academia (microbiology, genomics, biochemistry).

The invention may allow significant cost and time reductions in reagents and allow the optimisation of conditions for the growth of *inter alia* bacteria, fungi and mammalian cells.

### Description of the Preferred Embodiments

A device of the invention comprises a microfluidic chamber including a sensor and inlets for a sample and a growth medium, and preferably an outlet according to the claims. The reagents are preferably fed through one or more microfluidic channels into the chamber. An outlet of the chamber may then lead, preferably via a microfluidic channel, to a waste reservoir or another chamber. An output channel may be used as an overflow and sampling line and may be obstructed by a printed interdigitated pattern of closely spaced islands to act as a filter to prevent egress of the growing cells from the chamber.

A device of the invention is preferably formed using a plastics material. Plastics represent attractive materials for microfluidic systems since they are less expensive and more easily manipulated than silica/silicon-based substrates. A wide range of low-cost polymer materials enables selection of the appropriate plastics for thermal and chemical resistance, photolithographic and silk screen printing, surface derivation and multi-layer bonding. Plastics materials may include both linear and cross-linked, opaque and translucent plastics offering the possibility of inert surfaces for non-aqueous media, chemically-modifiable surfaces and devices which can be interrogated optically, or which may be electrically conducting. Compared to current glass, silica or silicon-based substrates, the use of plastics offers several distinct advantages including a feature resolution equivalent to silicon-based technologies, short design cycles, low tooling costs and substantial design flexibility, and the possibility of fabricating many thousands of devices on a single plastic sheet. Electrical connections can be made directly to the device itself, without the requirement for bonding, mounting or packaging technologies. Examples of suitable plastics materials include polyolefins such as polypropylene (PP), polycarbonate, PDMS, polyvinyl chloride (PVC), polystyrene, fluoropolymers and acrylates such as polymethyl metacrylate (PMMA).

Selection of appropriate materials should take into account the relevant characteristics of mechanical, chemical, electrical, acoustic, optical, gas permeability, surface morphology, sterilisability, biocompatibility, and, impotrantly, the ability to join, etch or print onto them. The potential effects of additives such as stabilisers, fillers, plasticisers and colourants, e.g. on cellular growth and viability, should also be taken into consideration.

A device of the invention may comprise a multi-layer construction. A material of the type described above is particularly suitable as a substrate.

Such a substrate may have microfluidic chambers/channels formed in it. Preferably, however, a different, more tractable material, such as an epoxy resin, is formed as a "matrix" layer on the substrate and the microfluidic chambers/channels are formed in that. If necessary or desired, the walls of these features may then be treated or coated, in order to inhibit cell attachment. The device may then be completed by providing a further layer of a substrate-type material, on top of the microfluidic-.defining layer.

Each layer may be formed and joined together by known techniques. Suitable formation techniques include printing and moulding; adhesive or lamination may be used to join layers. Similarly, the microfluidic may be formed by known microengineering procedures, including ablation using an excimer or other laser.

The substrate is preferably impermeable. In order to introduce samples/reagents etc, or to allow monitoring or the sensor, one or more holes may be punched or drilled in it, e.g. using a laser. Alternatively or in addition, the substrate material should allow a needle to be punched through it, through which materials are delivered.

The or each chamber typically has a volume of at least 50 nL, e.g. up to 10 µL but often no more than 500 nL, 1 µL or 2 µL. In a 500 µm thick substrate, holes of width 1.0, 1.5 and 2.0mm would result in chambers having a volume of approximately 400 nL, 900 nL and 1.6 µL, respectively.

A feature of the invention is that attachment of cells to a wall of the chamber is inhibited. By inhibiting the attachment of cells on the walls of the chamber, the formation of biofilm is controlled.

There are many methods of inhibiting cell attachment. These include coating the inside of a chamber and/or channels with a hydrophilic material, for example polyvinyl alcohol (PVA), and incorporating a non-metabolisable inhibitor of mannose-specific adhesin (e.g. methyl α-D-mannopyranoside) in the growth medium.

The wall of the chamber is preferably smooth. A substrate such as PMMA or PP is smooth, and the smoothness of an epoxy resin can be controlled, e.g. by sonication, degassing, or centrifugation before application. Further, treatment with acid or alkali can help to minimise nucleation points.

Bacterial growth may be promoted by increasing the supply of oxygen to the bioreactor chamber. The device preferably comprises an internal surface layer of a gas-permeable material, e.g. providing permeability to oxygen, CO₂ or NH₃. Alternatively, the internal surface may be oxygen-impermeable, for an (obligate) anaerobe. Examples of gas-permeable plastics include polyalkenes such as low-density polyethylene (LDPE) and polymethylpentene (PMP). These are also optically clear and chemically resistant. Polyalkenes are hydrophobic, "low-energy" plastics. For adhesion to an epoxy layer, some surface activation is necessary. It is also desirable to make the surface as hydrophilic as possible to prevent bacterial adhesion during use. This can be achieved by etching a polyalkene surface using an oxygen-plasma method; in this case, the polyalkene is preferably coated, e.g. with PVA, to prevent reversion to a hydrophobic surface. Alternatively, the surface of the polyalkene can be treated with chromic acid; this oxidises the surface of the plastic, rendering hydrophilicity. These techniques have been shown to reduce the adhesion of bacteria such as *E. coli* and *L. casei* by up to 90%.

A device of the invention preferably comprises a fluoropolymer layer. Fluoropolymers such as fluorinated ethylene-polypropylene (FEP) have excellent gas-permeability and optical properties. However, fluoropolymers have a low reactivity and thus require pre-treatment before application. One method is to use a reducing agent such as a sodium napthalide complex. This agent is available in a range of commercial mixes (e.g. Tetra-Etch produced by W. L. Gore Associates). Controlled reoxidation, e.g. using an oxidising agent such as nitric acid may be required.

The comprehensive investigation of biological processes demands an appreciation of both the biotic and abiotic phases. The biotic phase defines the cellular growth, metabolic and productivity parameters of a biological system, whilst the abiotic phase is defined by the products of metabolism which may be chemical and/or physical.

The device preferably comprises a biotic chamber in combination with an abiotic chamber. The abiotic chamber may comprise capture, separation and analytical systems designed to target individual profiles of products of the cellular process occurring in the biotic chamber. In this case, a bioreactor chamber may be viewed as a "quasi-chemostat", wherein the liquid overflow provides a sample source for downstream analysis of the products of metabolism. Patterns of metabolic response to nutritional and environmental stimuli may be investigated; these observations can then be used to make qualitative and quantitative judgements about the microorganism under investigation. The results may then be used to define the critical nutritional, environmental and temporal components.

Similarly, a device of the invention may also be used to capture specific proteins via appropriate affinity adsorbents or His-tag binding agents and monitoring their presence with a suitable sensor, for example a holographic or an acoustic sensor. The approach of using pulsed acoustic waves induced by magnetic direct generation is compatible with plastic substrates, since acoustic losses are considerably less than those experienced in the resonance format, and interrogation with a continuous spectrum of frequencies is possible, thereby allowing "fingerprinting" of target proteins, cells or oligonucleotides.

The design of the device may be tailored to its intended application. This may involve the provision of multi-channel feed inputs for dosing of more than one growth medium such as different carbon and nitrogen sources, vitamins, growth factors, mineral supplements and precursors, and additional sensors in the chamber. The chamber preferably comprises a stirrer, preferably a magnetic bead 20-100 µm in diameter. Other considerations include the printing and/or photolithographic etching of the microchamber, microchannels of high aspect ratio, microfluidic-driving mechanisms, flow characteristics, pumps, stirrers and retaining filters and the fabrication of downstream and analytical modules.

The chamber comprises one or more sensors for monitoring cellular activity, for example by detecting changes in biomass (cell numbers) or temperature and alterations in key attributes associated with growth (pH, dissolved oxygen, redox potential, substrate concentrations). A plurality of sensors may be located adjacent to each other in a control binnacle in the bioreactor chamber. The sensor(s) may be placed around the periphery of the well, thereby not only allowing a clear optical path length but also, in addition, acting as an actuator for a stirrer. A chamber may be fitted with a microscope (preferably equipped with a digital camera and imaging software) which may be used for monitoring eukaryotic cellular processes.

Holographic sensor technology is ideally suited to the present invention. A holographic sensor may be used to monitor a wide range of analytes, such as gases, ions, metabolites, antigens and whole cells, in real-time, with rapid response times. The sensor may be sensitive to combinations of analytes such as gases (e.g. O₂, CO₂), glucose, pH, lactate, glutamate/glutamine, temperature, redox, ions (e.g. ammonium ions) and cellular products (antibiotics, enzymes, expressed proteins). Suitable holograms of this type are described in WO-A-95/26499 and WO-A-99/63408.

Sensor electrodes can be used for the *in situ* measurement of pH, dissolved oxygen, glucose or other biochemical substrates. A pH-sensing electrode may comprise metal oxides such as tantalum pentoxide (printed from tantalum(V) isopropoxide, sol-gel formation and curing at 100-110°C) or the dioxides of iridium, platinum or ruthenium (printed from pre-sintered ruthenium dioxide hydrate mixed with a polymer paste in the ratio 1:2 by weight). A dissolved oxygen-sensing electrode may comprise a gold or three-layer construction comprising a substrate plastic, platinum microarray electrode and silver/silver bromide pseudo-reference, and a gas-permeable PTFE membrane enclosing an internal electrolyte gel. A temperature or redox potential may also be formed from printed platinum. Biomass may be measured by optical interrogation at 580-600 nm using an amber LED (γₘₐₓ 592nm) as a light source, a silicon photodiode/amplifier as detector and fibre optic light guides to convey the light into and out of the chamber. Further techniques that may be used include membrane fluorescence, colour change etc.

A ruthenium-based sensor may also be used, in particular a fluorescence quenching ruthenium-based oxygen sensor. A range of such compounds with a ruthenium ion and three phenanthroline-based ligands is available and can be immobilised in a polymer matrix for the purpose of determining oxygen content. Other fluorescence-based sensors can be used; there is a range of compounds giving increases or decreases in fluorescence with changes in ion concentrations, gas concentrations, pH etc. Similarly there are a range of simple colour change reactions that can be harnessed, especially for variables such as pH.

Data from the chamber can be stored on a datalogger, preferably comprising a computer installed with a suitable analogue interface. The interface circuitry is preferably flexible allowing simple high-quality, low-frequency measurements (e.g. every 15s over a 48 hour period) or for simultaneous "snapshots" of the signals (e.g. of over 400 sensors). The data produced by these circuits will be available to the computer such that datalogging, analysis and real-time feedback control systems. The efficient investigation and exploitation of biotic and abiotic processes using massively parallel cell culture systems requires the application of high-dimensionality data generation and analysis. Multi-factorial and other statistical methods are preferably incorporated for experimental design, control and analysis. Multivariate methods such as Principal Components Analysis can be used to derive information and knowledge from the holographic and other analytical response variable data sets. Algorithmic sensors and inferential engines may also be constructed combining disparate biotic and abiotic data streams to provide information and knowledge on metabolic, physiological and cellular status.

A device of the invention may be in the form of an array of microfluidic chambers, optionally connected by microchannels. The array may be an array of biotic and abiotic chambers. For example, such as array may be used to implement feed and management and control systems for monitoring the performance of up to a multiplicity of chambers, wherein each chamber on the array is provided with different growth media. Where necessary, careful consideration must be given to the configuration of the array to ensure that the microfluidic contacts and the scale-up issues associated with the macroscopic multiplexing of fluidics, optical interrogation systems and input/output contacts are achievable within the device. The array may be configured in a two-dimensional array, tape, disc or drum format, with any biotic, abiotic and analytical phases arranged appropriately. More generally, the sensors may be in the form of an array within one or more chambers of which any pair may be interconnected by a suitable channel. The layout of the array, e.g. in rows and columns, should merely be compatible with appropriate optical or other detection apparatus.

The present invention is particularly relevant to the study of cell culture, e.g. tissue culture and fermentation processes. Current technology for the study of fermentation development struggles to allow the simultaneous study of a large number of metabolic effectors, either singly or in combinations of interacting factors. This is necessary in order to define as rapidly as possible the fermentation protocol for maximum productivity. There is thus a need to devise a system that can simultaneously test hundreds or thousands of effectors of microbial physiology. This may be realised by using a device, preferably in the form of an array, of the invention.

The present invention may also be used in the study of filamentous microorganisms, biotransformation of precursor molecules, multiple culture inoculation (including spores), cell-cell signalling novel bioactive antibacterial compounds and metabolically engineered cells. Other applications of the present invention include the rapid analysis of mutationally segregate cells (in support of functional genomics), high-throughput screening of antibiotic production and susceptibility, automation of cellular bioassays for evaluating agonists/antagonists, cellular systems for the production of key biopharmaceutical compounds, measurement of cellular energy fluxes, metabolic and physiological status, mixed culture systems and their interactions, environmental impact of cytotoxic agents, receptor-mediated responses, subtypes and signal transduction pathways and ligand-gated ion channels.

The following Examples illustrate the invention.

### Example 1

Microfluidic devices were produced using a polycarbonate substrate printed with an epoxy resin. The resin layer was used to define input and output microchannels and the microfluidic chamber. Hydrochloric acid was introduced into the chamber of each device at a range of different concentrations (0.02 to 0.2M), and allowed to stand for 45 minutes to cure the epoxy groups. The devices were then rinsed with sterile distilled water until the pH of the rinse became the same as the background level of the water, and dried. A sample of *E. coli* cells and a growth medium was then introduced into the chamber and cultured.

Curing of the epoxy groups was found to significantly reduce the attachment of cells to the surface of the epoxy resin in the chamber, with little biofilm observed after 1 hour.

### Example 2

*E. coli* cells were cultured in non-agitated LB growth medium in three polycarbonate/epoxy resin microfluidic devices. One device comprised LB containing additional mannose whilst in another device the LB comprised the non-metabolisable mannose analogue, methyl α-mannopyranoside. After 4 hours incubation at 37°C, each device was stained with crystal violet and the number of bacteria attached per field of vision (at 600x magnification) counted.

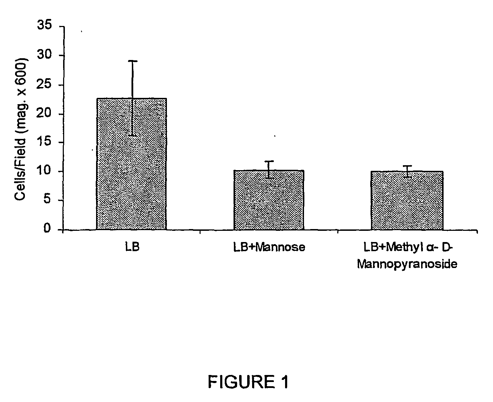

Figure 1 shows that the presence of mannose and the mannose analogue resulted in a significant reduction in the level of bacterial adherence.

### Example 3

A microfluidic device was formed by laminating a polycarbonate substrate and epoxy and LDPE layers. The LDPE provided means for introduction of a needle for material delivery. The formed epoxy was treated with acid and coating with polyvinyl alcohol (PVA), which was subsequently cross-linked with acidic glutaraldehyde. Excess glutaraldehyde was then washed out. Static *E. coli*/LB cultures were used to evaluate the degree of biofilm formation.

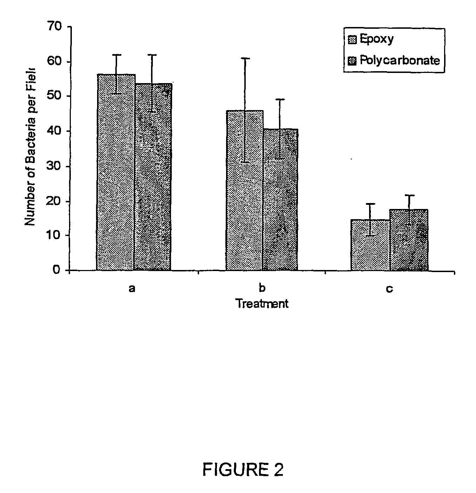

Figure 2 shows that the presence of the hydrophilic, cross-linked PVA significantly limits the extent of biofilm formation. For each pair of columns (a, b and c), the left column shows the bacterial count on the epoxy whilst the right column shows the count on the polycarbonate.

### Example 4

A range of polycarbonate-backed, epoxy-coated devices was prepared. Two-part epoxy was prepared using a standard mixing method and aliquots were sonicated to remove a portion of the air bubbles. After coating each device with PVA, the devices were separated according to the number of ruptured air bubbles present on the surface. Sonication was found to reduce the number of ruptured air bubbles by approximately 50%. The devices were then used to ferment static terrific broth cultures of *E. coli.*

Close investigation of the ruptured air bubbles revealed that whilst the majority of bubbles did not show any increase in the level of attached bacteria, some were covered by thick colonies of cells. These covered bubbles displayed distinctive "sharp" characteristics, with the edges distinct and pronounced. Approximately 5% of ruptured bubbles fell into this category.

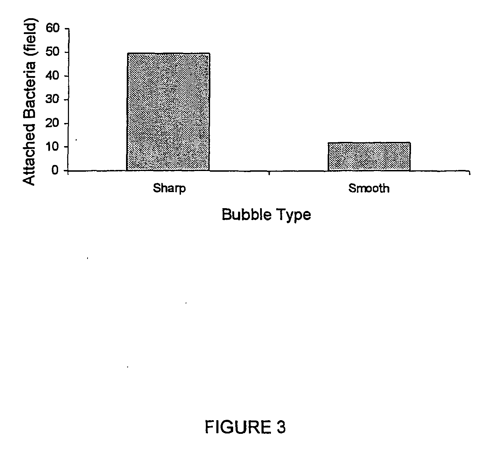

Figure 3 shows the number of bacteria attached to a polycarbonate/epoxy resin device having a "sharp" surface (produced by lamination with LDPE, which was removed once the epoxy was cured) and one having a "smooth" surface. The smooth surface is less susceptible to bacterial adhesion and thus biofilm formation.

### Example 5

Six polycarbonate-based microfluidic devices were produced comprising a polyalkene (LDPE) top coat. Two of the devices were treated with oxygen plasma for 30 seconds and another two for 90 seconds, to provide two sets of devices each treated to different extents. One set was then treated with PVA; the devices of the other set were not so treated. *E.coli* was then cultured in the chamber of each device.

Polyalkenes are hydrophobic and thus encourage bacterial adhesion. By coating the polyalkene with hydrophilic PVA, the bacterial count was expected to be reduced.

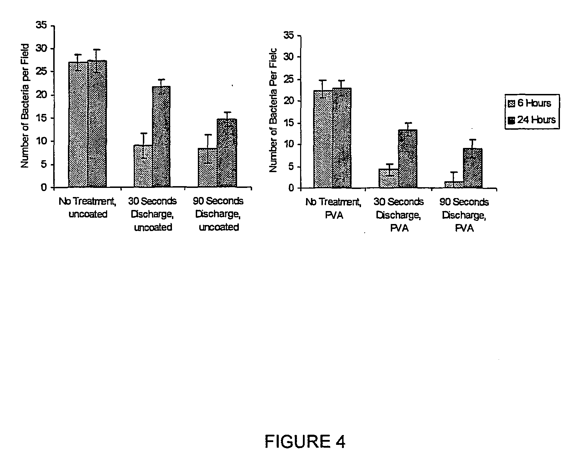

Figure 4 shows the initial bacteria count and that after 6 and 24 hours. Both plasma-treatment of the polyalkene and adding PVA reduce the level of bacterial adhesion.

### Example 6

Three polycarbonate/epoxy resin microfluidic devices were produced comprising a fluorinated ethylene polypropylene (FEP) upper layer. One of the devices was formed by pre-treating the FEP with sodium napthalide. Another of the devices was formed by pre-treating FEP with the naphthalene complex and coating with PVA. *E.coli* was then cultured in the microfluidic chamber of each device. Sodium naphthalene complex was used to increase the reactivity of FEP and thus aid production of the device.

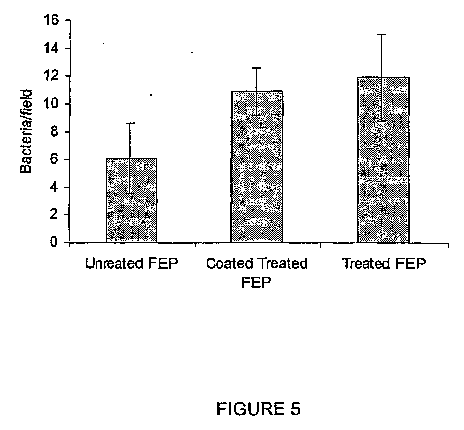

Figure 5 shows the bacterial count of each device. Whilst coating the FEP layer with sodium naphthalene complex does increase the number of adhered bacteria, the total number of adhered cells is still in the range found for other plastics materials typically used in a device of the invention. Coating the FEP layer with PVA caused a valuable reduction in bacterial adhesion. FEP is a preferred material due to its desirable gas-permeability and optical properties.

### Example 7

Two polycarbonate microfluidic devices were produced comprising oxygen-permeable top layers; one device comprised a polymethylpentene (TPX) top layer, the other an FEP top layer. *E.coli* was subsequently cultured in the microfluidic chamber of each device.

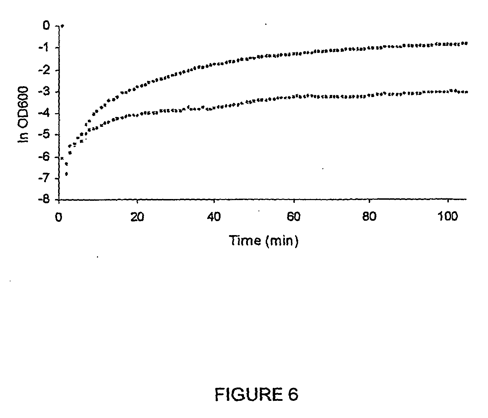

It was found that the rapid aerobic growth phase of the facultative anaerobe *E. coli* could be extended by using a more oxygen-permeable plastic. TPX is more permeable to oxygen than FEP and, as Figure 6 shows, supports a longer aerobic exponential growth phase before oxygen becomes a limiting nutrient.

## Claims

1. A method for monitoring cells in a microfluidic device, wherein the device includes a chamber having a volume of no more than 1 µL and comprising a sensor, and the monitoring is under conditions such that attachment of cells to the surface of the chamber is inhibited.

2. A method according to claim 1, wherein the chamber surface comprises a gas-permeable material.

3. A method according to claim 2, wherein the gas is CO₂, NH₃ or O₂.

4. A method according to claim 2 or claim 3, wherein the material is a fluoropolymer.

5. A method according to any preceding claim, wherein the chamber surface comprises a hydrophilic material.

6. A method according to claim 5, the hydrophilic material is polyvinyl alcohol.

7. A method according to any preceding claim, wherein the chamber is formed in an epoxy resin coated on a plastics substrate.

8. A method according to claim 7, wherein the plastics material is polycarbonate.

9. A method according to any preceding claim, wherein the chamber comprises a plurality of sensors.

10. A method according to any preceding claim, wherein the sensor is sensitive to oxygen, carbon dioxide, ammonium ion or pH.

11. A method according to any preceding claim, wherein the sensor is an optical sensor.

12. A method according to claim 11, wherein the sensor is a holographic sensor.

13. A method according to any of claims 1 to 10, wherein the sensor is an electrochemical or acoustic sensor.

14. A method according to any preceding claim, wherein the sensor is sensitive to a reactant or product of fermentation.

15. A method according to any preceding claim, wherein the volume of the chamber is from 50 nL to 1 µL.

16. A method according to any preceding claim, which further comprises introducing growth medium into the chamber, wherein the sensor is sensitive to a reactant or product of cell growth.

17. A method according to claim 16, wherein the growth medium comprises a non-metabolisable mannose analogue.

18. A method according to claim 17, wherein the analogue is methyl α-D-mannopyranoside.

19. A method according to any preceding claim, which further comprises introducing a component of or derived from the cells into a second microfluidic chamber comprising a sensor and in connection with the first chamber detecting said component.

20. A method according to claim 19, wherein the component is a product of cell growth.

21. A method according to claim 19, wherein the component is an expressed protein or enzyme.

22. A method according to any of claims 19 to 21, wherein the sensor of the second chamber is as defined in any of claims 10 to 15.

23. A microfluidic device suitable for use in a method according to any preceding claim, which comprises a chamber having a volume of no more than 1 µL and including a sensor and inlets for a sample and for a growth medium, wherein the chamber surface is such that, in use, attachment of cells thereto is inhibited.

24. A device according to claim 23, having any of the features defined in claims 2 to 15.

25. A device according to claim 23 or claim 24, which comprises a plurality of the chambers.

26. A device according to claim 25, wherein the chambers are in the form of an array.

27. A device according to claim 25 or claim 26, wherein a pair of chambers is connected by a channel.

## Patentansprüche

1. Verfahren zur Überwachung von Zellen in einer Mikrofluidvorrichtung, wobei die Vorrichtung eine Kammer umfasst, die ein Volumen von nicht mehr als 1 µl aufweist und einen Sensor umfasst, und wobei das Überwachen unter solchen Bedingungen erfolgt, dass das Anhaften von Zellen an der Oberfläche der Kammer gehemmt wird.

2. Verfahren nach Anspruch 1, wobei die Kammeroberfläche ein gasdurchlässiges Material umfasst.

3. Verfahren nach Anspruch 2, wobei es sich bei dem Gas um CO₂, NH₃ oder O₂ handelt.

4. Verfahren nach Anspruch 2 oder Anspruch 3, wobei es sich beim Material um ein Fluorpolymer handelt.

5. Verfahren nach einem der vorstehenden Ansprüche, wobei die Kammeroberfläche ein hydrophiles Material umfasst.

6. Verfahren nach Anspruch 5, wobei es sich beim hydrophilen Material um Polyvinylalkohol handelt.

7. Verfahren nach einem der vorstehenden Ansprüche, wobei die Kammer aus einem Epoxyharz gebildet ist, das schichtförmig auf ein Kunststoffsubstrat aufgebracht ist.

8. Verfahren nach Anspruch 7, wobei es sich beim Kunststoffmaterial um Polycarbonat handelt.

9. Verfahren nach einem der vorstehenden Ansprüche, wobei die Kammer eine Mehrzahl von Sensoren umfasst.

10. Verfahren nach einem der vorstehenden Ansprüche, wobei der Sensor gegenüber Sauerstoff, Kohlendioxid, Ammoniumionen oder dem pH-Wert empfindlich ist.

11. Verfahren nach einem der vorstehenden Ansprüche, wobei es sich beim Sensor um einen optischen Sensor handelt.

12. Verfahren nach Anspruch 11, wobei es sich beim Sensor um einen holographischen Sensor handelt.

13. Verfahren nach einem der Ansprüche 1 bis 10, wobei es sich beim Sensor um einen elektrochemischen oder akustischen Sensor handelt.

14. Verfahren nach einem der vorstehenden Ansprüche, wobei der Sensor gegenüber einem Reaktanten oder Produkt einer Fermentation empfindlich ist.

15. Verfahren nach einem der vorstehenden Ansprüche, wobei das Volumen der Kammer 50 nl bis 1 µl beträgt.

16. Verfahren nach einem der vorstehenden Ansprüche, ferner umfassend das Zuführen eines Wachstumsmediums in die Kammer, wobei der Sensor gegenüber einem Reaktanten oder einem Produkt des Zellwachstums empfindlich ist.

17. Verfahren nach Anspruch 16, wobei das Wachstumsmedium ein nichtmetabolisierbares Mannose-Analogon umfasst.

18. Verfahren nach Anspruch 17, wobei es sich beim Analogon um Methyl-α-D-mannopyranosid handelt.

19. Verfahren nach einem der vorstehenden Ansprüche, ferner umfassend das Zuführen einer Komponente von oder abgeleitet von den Zellen in eine zweite Mikrofluidkammer, die einen Sensor umfasst und in Verbindung mit der ersten Kammer, die die Komponente nachweist, steht.

20. Verfahren nach Anspruch 19, wobei es sich bei der Komponente um ein Produkt des Zellwachstums handelt.

21. Verfahren nach Anspruch 19, wobei es sich bei der Komponente um ein exprimiertes Protein oder Enzym handelt.

22. Verfahren nach einem der Ansprüche 19 bis 21, wobei der Sensor der zweiten Kammer wie in einem der Ansprüche 10 bis 15 definiert ist.

23. Mikrofluidvorrichtung, die sich zur Verwendung in einem Verfahren nach einem der vorstehenden Ansprüche eignet, umfassend eine Kammer mit einem Volumen von nicht mehr als 1 µl, die einen Sensor und Einlässe für eine Probe und für ein Wachstumsmedium aufweist, wobei die Kammeroberfläche so beschaffen ist, dass bei der Verwendung ein Anhaften von Zellen daran gehemmt wird.

24. Vorrichtung nach Anspruch 23, die eines der Merkmale gemäß der Definition in den Ansprüchen 2 bis 15 umfasst.

25. Vorrichtung nach Anspruch 23 oder 24, umfassend eine Mehrzahl von den Kammern.

26. Vorrichtung nach Anspruch 25, wobei die Kammern in Form eines Arrays vorliegen.

27. Vorrichtung nach Anspruch 25 oder Anspruch 26, wobei ein Paar von Kammern durch einen Kanal verbunden ist.

## Revendications

1. Procédé pour contrôler des cellules dans un dispositif microfluidique, dans lequel le dispositif comprend une chambre ayant un volume non supérieur à 1 µl et comprenant un capteur, et le contrôle est effectuée dans des conditions telles que la fixation des cellules à la surface de la chambre est inhibée.

2. Procédé suivant la revendication 1, dans lequel la surface de la chambre comprend une matière perméable aux gaz.

3. Procédé suivant la revendication 2, dans lequel le gaz est CO₂, NH₃ ou O₂.

4. Procédé suivant la revendication 2 ou la revendication 3, dans lequel la matière est un polymère fluoré.

5. Procédé suivant l'une quelconque des revendications précédentes, dans lequel la surface de la chambre comprend une matière hydrophile.

6. Procédé suivant la revendication 5, dans lequel la matière hydrophile est l'alcool polyvinylique.

7. Procédé suivant l'une quelconque des revendications précédentes, dans lequel la chambre est formée dans une résine époxy revêtant un substrat en matière plastique.

8. Procédé suivant la revendication 7, dans lequel la matière plastique est un polycarbonate.

9. Procédé suivant l'une quelconque des revendications précédentes, dans lequel la chambre comprend une pluralité de capteurs.

10. Procédé suivant l'une quelconque des revendications précédentes, dans lequel le capteur est sensible à l'oxygène, au dioxyde de carbone, à l'ion ammonium ou au pH.

11. Procédé suivant l'une quelconque des revendications précédentes, dans lequel le capteur est un capteur optique.

12. Procédé suivant la revendication 11, dans lequel le capteur est un capteur holographique.

13. Procédé suivant l'une quelconque des revendications 1 à 10, dans lequel le capteur est un capteur électrochimique ou acoustique.

14. Procédé suivant l'une quelconque des revendications précédentes, dans lequel le capteur est sensible à un corps réactionnel ou produit de fermentation.

15. Procédé suivant l'une quelconque des revendications précédentes, dans lequel le volume de la chambre est compris dans l'intervalle de 50 nl à 1 µl.

16. Procédé suivant l'une quelconque des revendications précédentes, qui comprend en outre l'introduction d'un milieu de croissance dans la chambre, dans lequel le capteur est sensible à un corps réactionnel ou produit de la croissance des cellules.

17. Procédé suivant la revendication 16, dans lequel le milieu de croissance comprend un analogue du mannose non métabolisable.

18. Procédé suivant la revendication 17, dans lequel l'analogue est le méthyl-α-D-mannopyranoside.

19. Procédé suivant l'une quelconque des revendications précédentes, qui comprend en outre l'introduction d'un constituant des, ou dérivés des, cellules dans une seconde chambre microfluidique comprenant un capteur et, en connexion avec la première chambre, la détection dudit constituant.

20. Procédé suivant la revendication 19, dans lequel le constituant est un produit de la croissance des cellules.

21. Procédé suivant la revendication 19, dans lequel le constituant est une protéine ou enzyme exprimée.

22. Procédé suivant l'une quelconque des revendications 19 à 21, dans lequel le capteur de la seconde chambre est tel que défini dans l'une quelconque des revendications 10 à 15.

23. Dispositif microfluidique apte à l'utilisation dans un procédé suivant l'une quelconque des revendications précédentes, qui comprend une chambre ayant un volume non supérieur à 1 µl et comprenant un capteur et des orifices d'admission pour un échantillon et pour un milieu de croissance, dans lequel la surface de la chambre est telle que, lors de l'utilisation, la fixation des cellules à celle-ci est inhibée.

24. Dispositif suivant la revendication 23, ayant n'importe lesquelles des caractéristiques définies dans les revendications 2 à 15.

25. Dispositif suivant la revendication 23 ou la revendication 24, qui comprend une pluralité des chambres.

26. Dispositif suivant la revendication 25, dans lequel les chambres sont sous forme d'un réseau.

27. Dispositif suivant la revendication 25 ou la revendication 26, dans lequel une paire de chambres est connectée par un canal.
